# EUROPÄISCHE PATENTSCHRIFT

(11) **EP 2 424 831 B1**
(45) Veröffentlichungstag und Bekanntmachung des Hinweises auf die Patenterteilung: **14.08.2013**
(21) Anmeldenummer: 10714315.8
(22) Anmeldetag: 22.04.2010
(51) Int. Cl.: C07C 67/055, C07C 69/15

(54) **VERFAHREN ZUR HERSTELLUNG VON VINYLACETAT**
METHOD FOR PRODUCING VINYL ACETATE
PROCÉDÉ DE FABRICATION D'ACÉTATE DE VINYLE

(30) Priorität: 27.04.2009 DE 102009002666
(43) Veröffentlichungstag der Anmeldung: 07.03.2012
(73) Patentinhaber: Wacker Chemie AG, 81737 München (DE)
(72) Erfinder: DAFINGER, Willibald, 94133 Röhrnbach (DE); HOLL, Peter, 84547 Emmerting (DE); WAGNER, Johann, 84489 Burghausen (DE)
(74) Vertreter: Schuderer, Michael
(86) Internationale Anmeldenummer: PCT/EP2010/055348
(87) Internationale Veröffentlichungsnummer: WO 2010/124985

(56) Entgegenhaltungen:
- EP-A2- 1 642 882
- WO-A1-2005/100296
- DE-A1-102006 038 689

## Beschreibung

Die Erfindung betrifft ein Verfahren zur Herstellung von Vinylacetat in einem heterogen katalysierten, kontinuierlichen Gasphasenprozess durch Umsetzung von Ethylen mit Essigsäure und Sauerstoff in einem Reaktor, wobei die Aufarbeitung des dabei erhaltenen Produktstromes energetisch optimiert wird.

Vinylacetat wird in kontinuierlichen Verfahren unter Rückführung des aufgereinigten Produktstromes hergestellt (Kreisgas-System). Dabei reagiert in einem heterogen katalysierten Gasphasenprozess Ethylen mit Essigsäure und Sauerstoff an Festbett-Katalysatoren, welche im allgemeinen Palladium- und Alkalimetallsalze auf einem Trägermaterial enthalten und zusätzlich noch mit Gold, Rhodium oder Cadmium dotiert sein können.

Die Edukte Ethylen, Sauerstoff und Essigsäure werden in einer exothermen Reaktion im allgemeinen bei einem Druck von 1 bis 30 bar und einer Temperatur von 130°C bis 200°C in einem Festbett-Röhrenreaktor (oder auch Wirbelschichtreaktor) zu Vinylacetat umgesetzt: C₂H₄ + CH₃COOH + ½ O₂ -> CH₃COOCH=CH₂ + H₂O In der Nebenreaktion wird Ethylen zu CO₂ oxidiert:

C₂H₄ + 3 O₂ → 2 CO₂ + 2 H₂O

Bei der Herstellung von Vinylacetat wird ein überwiegend aus Ethylen, Kohlendioxid, Ethan, Stickstoff und Sauerstoff bestehendes Gasgemisch im Kreis geführt. Der Gasstrom wird vor dem Festbettröhrenreaktor mit den Reaktanden Essigsäure, Ethylen und Sauerstoff versetzt und mit Heizdampf betriebenen Wärmetauschern auf Reaktionstemperatur gebracht. Die Anreicherung des Kreisgases mit Essigsäure erfolgt vor dem Festbettröhrenreaktor, üblicherweise mittels einem mit Heizdampf betriebenen Essigsäuresättiger (Essigsäureverdampfer).

Der aus dem Reaktor austretende Produktgasstrom enthält im Wesentlichen Vinylacetat, Ethylen, Essigsäure, Wasser, Sauerstoff, CO₂ sowie die Inerten Stickstoff, Argon, Methan und Ethan. Nach der Reaktion werden das Reaktionsprodukt Vinylacetat, nicht umgesetzte Essigsäure, Wasser und weitere kondensierbare Anteile aus dem Kreisgas auskondensiert und der Aufarbeitung zugeführt. Nicht auskondensiertes Vinylacetat wird in einem mit Essigsäure betriebenen Wäscher ausgewaschen. Die auskondensierten Produkte Vinylacetat und Wasser sowie nicht umgesetzte Essigsäure werden in verschiedenen, üblicherweise mit Heizdampf betriebenen, Destillationsprozessen voneinander getrennt und das verbleibende, Ethylen enthaltende, Kreisgas, nach Verdichtung, zurückgeführt.

Ein Teilstrom des Kreisgases wird, vor der Rückführung in den Reaktor, auf der Druckseite des Kreisgasverdichters abgezweigt, zur CO₂-Entfernung der CO₂-Wäsche (CO₂-Absorption/Desorption) zugeführt und nach der CO₂-Entfernung wieder saugseitig zum Verdichter zurückgeführt. Vor der CO₂-Entfernung wird dieser Teilstrom üblicherweise in einer Kolonne (Wasserwäscher) mittels Zugabe von Essigsäure und Wasser von Vinylacetat befreit. Das Sumpfprodukt dieser Kolonne, welches Wasser, Essigsäure und VAM enthält, wird in der Azeotropkolonne, welche mit Prozessdampf beheizt wird, aufgetrennt.

Problematisch bei den Prozeßschritten zur CO₂-Wäsche ist die Notwendigkeit den Teilstrom, aufgrund der bei der Wäsche auftretenden Druckverluste saugseitig zurückzuführen, mit dem Nachteil, dass der gesamte Teilstrom nochmals verdichtet werden muß (zusätzliche Belastung Verdichter). Die saugseitige Rückführung bedingt zudem, dass nach der Verdichtung der bereits aufgereinigte Teilstrom wieder dem ungereinigten Kreisgasstrom zugeführt wird und teilweise wieder der CO₂-Wäsche zugeführt wird.

Ein weiterer Nachteil der bisher praktizierten Prozeßschritte zur CO₂-Wäsche ist der Energieaufwand zur Auftrennung des nach der Wäsche im Kreisgaswäscher anfallenden Rohvinylacetats in einer Azeotropkolonne.

Vor diesem Hintergrund bestand die Ausgabe, die Aufarbeitung des zur CO₂-Wäsche abgezweigten Teilstroms energetisch zu optimieren.

Aus der DE 10 2006 038 689 A1 ist bekannt, den Wärmeinhalt des bei der CO₂-Wäsche aus dem Kohlendioxid-Desorber erhaltenen Wasserdampf-gesättigten CO₂-Stroms zur Aufheizung des Sumpfes der Reinvinylacetatkolonne einzusetzen. Nachteilig ist dabei der schlechte Wärmeübergang bei Einsatz eines gasförmigen Wärmeübertragungsmediums. Weiter ist der Wärmeinhalt dieses CO₂-Stromes, aufgrund des relativ niederen Anteils an Wasserdampf, sehr gering.

Gegenstand der Erfindung ist ein Verfahren zur Herstellung von Vinylacetat in einem heterogen katalysierten, kontinuierlichen Gasphasenprozess durch Umsetzung von Ethylen mit Essigsäure und Sauerstoff in einem Reaktor, und
a) Auftrennung des Produktgasstromes enthaltend im Wesentlichen Ethylen, Vinylacetat, Essigsäure, Wasser, Kohlendioxid und weitere Inertgase, und
b) Rückführung eines Ethylen und CO₂ enthaltenden Kreisgasstromes in den Reaktor, wobei
c) der Kreisgasstrom vor Rückführung in den Reaktor in einem Kreisgasverdichter, verdichtet wird, und
d) ein Teilstrom des Kreisgases auf der Saugseite oder der Druckseite des Kreisgasverdichters abgezweigt und einer CO₂-Wäsche zugeführt wird, und
e) vor der CO₂-Wäsche in einem Wasserwäscher gewaschen wird, dadurch gekennzeichnet, dass
f) der Teilstrom nach der CO₂-Wäsche über einen Strahlverdichter, zusammen mit Ethylen als Treibmittel, auf der Druckseite des Kreisgasverdichters und stromabwärts von der Entnahme des Teilstroms zur CO₂-Wäsche dem Kreisgas zugeführt wird,

Bei der kontinuierlichen Herstellung von Vinylacetat wird vorzugsweise in Röhrenreaktoren gearbeitet, welche mit einem Festbettkatalysator beschickt sind. Diese Katalysatoren sind im allgemeinen mit Edelmetall(salz)en und Promotoren dotierte Trägerkatalysatoren, beispielsweise mit Palladium und mit Gold und Kalium-Salzen dotierte Bentonit-Kugeln. Der Reaktor wird mit Ethylen, Sauerstoff und Essigsäure beschickt und die Reaktion bei einem Druck von vorzugsweise 8 bis 12 bar abs. und einer Temperatur von vorzugsweise 130°C bis 200°C durchgeführt.

Die Reaktionstemperatur im Festbettröhrenreaktor von vorzugsweise 130°C bis 200°C wird mittels Siedewasserkühlung bei einem Druck von 1 bis 30 bar abs. eingestellt. Dabei wird Wasserdampf, der sogenannte Eigendampf, mit einer Temperatur von 120°C bis 185°C bei einem Druck von 1 bis 10 bar abs., vorzugsweise 2,5 bis 5 bar abs., gebildet. Der aus dem Reaktor austretende Produktgasstrom enthält im Wesentlichen Vinylacetat, Ethylen, Essigsäure, Wasser, Sauerstoff, CO₂ sowie die Inerten Stickstoff, Argon, Methan und Ethan.

Im Allgemeinen wird das den Festbettröhrenreaktor verlassende Gasgemisch in eine Vorentwässerungskolonne geleitet und die am Sumpf der Kolonne anfallende flüssige Phase, hauptsächlich Vinylacetat, Essigsäure, Ethylacetat und Wasser, dem Rohvinylacetat-Sammelbehälter zugeführt. In der nachgeschalteten Azeotropkolonne wird in Vinylacetatmonomer (VAM) und Wasser als Kopfprodukt und Essigsäure als Sumpfprodukt aufgetrennt. Die Essigsäure wird in den Essigsäuresättiger geleitet und so in den Prozess zurückgeführt. Das als Kopfprodukt entnommene VAM wird über die Entwässerungskolonne zur Reinvinylacetatkolonne geleitet und dort in VAM und Essigsäure getrennt.

Das am Kopf der Vorentwässerungskolonne entnommene gasförmige Produktgemisch, bestehend im Wesentlichen aus Ethylen und CO₂, wird im Kreisgaswäscher von allen kondensierbaren Anteilen befreit. Der am Kopf des Kreisgaswäschers entnommene Kreisgasstrom wird im Kreisgasverdichter, zum Ausgleich der im Reaktionskreislauf auftretenden, beträchtlichen Druckverluste, verdichtet und wieder als Kreisgas in den Reaktor zurückgeführt. Das Druckniveau des Kreisgases beträgt vorzugsweise 8 bis 12 bar abs. Nach der Abtrennung der kondensierbaren Anteile Vinylacetat, Essigsäure und Wasser im Kreisgaswäscher enthält das Kreisgas vorzugsweise 12 bis 18 Vol.-% CO₂.

Ein Teilstrom des Kreisgases wird auf der Saugseite oder Druckseite, vorzugsweise der Druckseite, des Kreisgasverdichters abgezweigt und zur CO₂-Entfernung der CO₂-Wäsche zugeführt und geht nach der CO₂-Entfernung wieder druckseitig zurück zum Verdichter. Der am Kreisgasverdichter entnommene Teilstrom macht etwa 8 bis 12 Vol.-% des gesamten Kreisgases aus. Nach der Abzweigung und vor der CO₂-Wäsche wird der entnommene Teilstrom in einer Kolonne (Wasserwäscher) unter Zuführung von Wasser und Essigsäure gewaschen. Das flüssige Sumpfprodukt kann im Rohvinylacetatbehälter gesammelt werden und in der nachgeschalteten Azeotropkolonne aufgetrennt werden. Das gasförmige Kopfprodukt des Wasserwäschers, im Wesentlichen Ethylen und CO₂, wird der CO₂-Wäsche zugeführt.

Der Kreisgasteilstrom (Kopfprodukt des Wasserwäschers) wird nun in eine, vorzugsweise mit wässriger Kaliumcarbonat-Lösung betriebene, CO₂-Absorption/Desorption geführt. Nach der CO₂-Wäsche enthält der Kreisgasteilstrom vorzugsweise noch 2 bis 6 Vol.-% CO₂. Der Druckverlust nach Kreisgaswäsche und CO₂-Absorption/Desorption beträgt im Allgemeinen 2 bis 4 bar.

Der Kreisgasteilstrom wird nach der CO₂-Wäsche, auf der Druckseite des Kreisgasverdichters, und stromabwärts von der Entnahmestelle des Teilstroms zur CO₂-Wäsche, in den Kreisgastrom zurückgeführt. Zum Ausgleich des Druckverlustes wird der Kreisgasteilstrom mittels Ethylen, welches aus der Raffinerie mit einem Druck von 20 bis 25 bar abs. anfällt, auf ein Druckniveau von vorzugsweise 0,5 bis 2 bar über dem Druck des Kreisgas gebracht, und dem Kreisgasstrom zugeführt. Vorzugsweise wird der Kreisgasteilstrom mit der erforderlichen Menge Ethylen über einen Strahlverdichter (Ejektoren, Injektoren), vorzugsweise eine Saugdüse, zugeführt. In einer bevorzugten Ausführungsform kann auch so vorgegangen werden, dass der gesamte Ethylenfeed, welcher vor dem Reaktor dem Kreisgas zugeführt wird, über den Strahlverdichter zugeführt wird. Zusätzlich zu dieser Verfahrensweise der Rückführung des Kreisgasteilstroms kann auch so vorgegangen werden, dass der Betrieb des Wasserwäschers vor der CO₂-Absorption/Desorption erfindungsgemäß umgestaltet wird. Der Wasserwäscher wird vorzugsweise bei einem Druck von 8 bis 12 abs. und einer Temperatur von vorzugsweise 18 bis 30°C betrieben. Der Kreisgasteilstrom wird im Wasserwäscher mittels Essigsäure von VAM befreit und mittels Wasser von mitgerissener Essigsäure befreit. Am Kopf wird der gasförmige Restanteil des Kreisgasteilstroms entnommen und zur CO₂-Wäsche geführt.

Bei der erfindungsgemäßen Modifikation wird die am Sumpf des Wasserwäschers anfallende flüssige Phase, hauptsächlich Vinylacetat, Essigsäure, Ethylacetat und Wasser nicht mehr dem Rohvinylacetat-Sammelbehälter zugeführt und in der Azeotropkolonne aufgetrennt, sondern direkt der Vorentwässerungskolonne aufgegeben. Vorzugsweise wird das Sumpfprodukt dabei durch Wärmetausch mit dem aus dem Reaktor austretenden gasförmigen Produktstrom erwärmt. In der Vorentwässerungskolonne wird dann ohne externen Dampfeinsatz ein VAM-Wasser-Azeotrop erhalten und anschließend in einem Phasentrenner separiert. Die mit dieser Maßnahme zu erzielende Dampfeinsparung für die Entfernung des dem Wasserwäscher zugeführten Wassers liegt bei einem Gewichtsverhältnis von Dampf/Wasser von etwa 2 : 1. Mittels dieser Maßnahme wird in einer großtechnischen Anlage bei einem Wasserstrom von 300 bis 500 kg Wasser/Stunde eine Dampfeinsparung von etwa 1 to/h an der Azeotropkolonne erzielt.

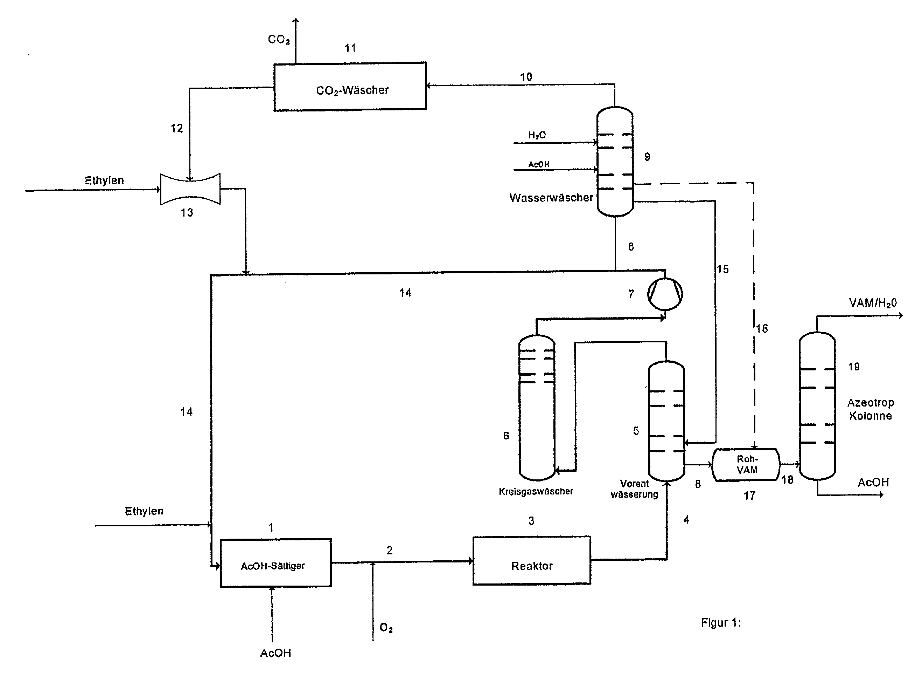

Figur 1 zeigt eine vereinfachte Darstellung des Prozesses: Ethylenhaltiges Kreisgas wurde im Essigsäuresättiger 1 mit Essigsäure beschickt, danach Sauerstoff hinzugefügt und über eine mit Dampf beheizte Leitung 2 dem Röhrenreaktor 3 zugeführt. Das den Reaktor verlassende Kreisgas-Gemisch, welches im Wesentlichen Ethylen, Vinylacetat, Essigsäure, Kohlendioxid, Sauerstoff sowie Inerte enthielt, wurde über Leitung 4 der Vorentwässerungskolonne 5 zugeführt. In der Vorentwässerungskolonne 5 wurde das Gemisch aufgetrennt, wobei das Sumpfprodukt mit im Wesentlichen VAM, Essigsäure und Wasser über Leitung 8 dem Rohvinylacetat-Behälter 17 zugeführt wurde, und nach Transfer über Leitung 18 in die Azeotropkolonne 19 in eine VAM-Fraktion und eine Essigsäure-Fraktion aufgetrennt wurde, welche jeweils, in hier nicht dargestellten Prozeßschritten weiter aufgearbeitet wurden.

Das Kopfprodukt der Vorentwässerungskolonne 5 wurde entnommen und im nachgeschalteten Kreisgaswäscher 6 mittels Waschen mit Essigsäure von gasförmigem VAM befreit. Das Gasgemisch (Kreisgas) bestand nach der Kreisgaswäsche im Wesentlichen aus Ethylen mit einem etwa 12 Vol.-% umfassenden CO₂-Anteil, und wurde mit dem Kreisgas-Verdichter 7 um etwa 3 bar höherverdichtet. Der Großteil des Kreisgases wurde über Leitung 14 in den Essigsäuresättiger 1 zurückgeführt.

Ein Anteil von etwa 12 Vol.-% des Kreisgases wurde druckseitig vom Kreisgasverdichter 7 abgezweigt und über Leitung 8 in den Wasserwäscher 9 überführt und dort, zur Entfernung von restlichem Vinylacetat, mit Essigsäure und anschließend Wasser behandelt. Das Essigsäure, Wasser und Vinylacetat umfassende Sumpfprodukt wurde über Leitung 15 direkt in die Vorentwässerungskolonne 5 eingeleitet. (Die gestrichelte Leitung 16 zeigt die im Stand der Technik übliche Ausführungsform bei der das Sumpfprodukt des Wasserwäschers 9 in den Rohvinylacetat-Behälter 17 überführt wird und anschließend in der AzeotropKolonne 19 aufgetrennt wird.)

Das Kopfprodukt des Wasserwäschers 9 wurde über Leitung 10 in den CO₂-Wäscher 11 überführt, welcher in herkömmlicher Weise über eine mit Kaliumcarbonat befüllte Absorptionseinheit und einen Desorber verfügt. Nach der CO₂-Wäsche hatte der Kreisgasteilstrom einen CO₂-Gehalt von 2 Vol.-% und einen Druck von 9 bis 11 bar abs.. Vor der Rückführung über Leitung 12 in die Kreisgasleitung 14 wurden über einen Strahlsauger 13 der gesamte Ethylenfeed mit einem Druck von 20 bis 25 bar abs. eingesaugt.

Die Energieeinsparung am Kreisgasverdichter 7 betrug ca. 1000 MWh pro Jahr.

## Patentansprüche

1. Verfahren zur Herstellung von Vinylacetat in einem heterogen katalysierten, kontinuierlichen Gasphasenprozess durch Umsetzung von Ethylen mit Essigsäure und Sauerstoff in einem Reaktor, und
a) Auftrennung des Produktgasstromes enthaltend im Wesentlichen Ethylen, Vinylacetat Essigsäure, Wasser, Kohlendioxid und weitere Inertgase in verschiedenen Destillationsprozessen enthaltend eine Vorentwässerungskolonne, einen Kreisgaswäscher und eine Azeotropkolonne und
b) Rückführung eines Ethylen und CO₂ enthaltenden Kreisgasstromes in den Reaktor, wobei
c) der Kreisgasstrom vor Rückführung in den Reaktor in einem Kreisgasverdichter, verdichtet wird, und
d) ein Teilstrom des Kreisgases auf der Saugseite oder der Druckseite des Kreisgasverdichters abgezweigt und einer CO₂-Wäsche zugeführt wird, und
e) vor der CO₂-Wäsche in einem Wasserwäscher gewaschen wird,
**dadurch gekennzeichnet, dass**
f) der Teilstrom nach der CO₂-Wäsche über einen Strahlverdichter, zusammen mit Ethylen als Treibmittel, auf der Druckseite des Kreisgasverdichters und stromabwärts von der Entnahme des Teilstroms zur CO₂-Wäsche dem Kreisgas zugeführt wird

2. Verfahren nach Anspruch 1, **dadurch gekennzeichnet, dass**
g) das Sumpfprodukt aus dem Wasserwäscher direkt der Vorentwässerungskolonne zugeführt wird.

3. Verfahren nach Anspruch 1 oder 2, **dadurch gekennzeichnet, dass** der gesamte Ethylenfeed, welcher vor dem Reaktor dem Kreisgasstrom zugeführt wird, über den Strahlverdichter zugeführt wird.

4. Verfahren nach Anspruch 1 bis 3, **dadurch gekennzeichnet, dass** das Sumpfprodukt aus dem Wasserwäscher mittels Wärmetausch mit dem Kreisgas erhitzt wird und der Vorentwässerungskolonne zugeführt wird.

## Claims

1. Process for preparing vinyl acetate monomer in a heterogeneously catalysed, continuous gas phase process by reacting ethylene with acetic acid and oxygen in a reactor, and
a) separating the product gas stream comprising essentially ethylene, vinyl acetate monomer, acetic acid, water, carbon dioxide and further inert gases in different distillation processes comprising a preliminary dewatering column, a cycle gas scrubber and an azeotrope column, and
b) recycling an ethylene- and CO₂-containing cycle gas stream into the reactor, by
c) compressing the cycle gas stream in a cycle gas compressor before recycling into the reactor, and
d) branching off a substream of the cycle gas on the suction side or the pressure side of the cycle gas compressor and feeding it to a CO₂ scrubbing, and
e) prior to the CO₂ scrubbing, washing it in a water scrubber,
**characterized in that**
f) the substream, after the CO₂ scrubbing, is supplied to the cycle gas via a jet compressor, together with ethylene as the motive medium, on the pressure side of the cycle gas compressor and downstream of the withdrawal of the substream to the CO₂ scrubbing.

2. Process according to Claim 1, **characterized in that**
g) the bottom product from the water scrubber is fed directly to the preliminary dewatering column.

3. Process according to Claim 1 or 2, **characterized in that** the entire ethylene feed which is supplied to the cycle gas stream upstream of the reactor is supplied via the jet compressor.

4. Process according to Claims 1 to 3, **characterized in that** the bottom product from the water scrubber is heated by means of heat exchange with the cycle gas and is fed to the preliminary dewatering column.

## Revendications

1. Procédé pour la préparation d'acétate de vinyle dans un processus continu en phase gazeuse, catalysé par voie hétérogène, par transformation d'éthylène avec de l'acide acétique et de l'oxygène dans un réacteur, et
a) séparation du flux de gaz produit contenant essentiellement de l'éthylène, de l'acétate de vinyle, de l'acide acétique, de l'eau, du dioxyde de carbone et d'autres gaz inertes dans différents procédés de distillation contenant une colonne de prédéshydratation, un laveur de gaz en circulation et une colonne à azéotrope et
b) recyclage d'un flux de gaz en circulation contenant de l'éthylène et du CO₂ dans le réacteur,
c) le flux de gaz en circulation étant compressé dans le réacteur dans un compresseur de gaz en circulation et
d) un flux partiel du gaz en circulation étant dévié côté aspiration ou côté pression du compresseur de gaz en circulation et introduit dans un lavage de CO₂, et
e) étant lavé dans un laveur d'eau avant le lavage de CO₂,
**caractérisé en ce que**
f) le flux partiel après le lavage de CO₂ est alimenté dans le gaz en circulation, via un compresseur à jets, ensemble avec l'éthylène comme gaz propulseur, du côté pression du compresseur de gaz en circulation et en aval du prélèvement du flux partiel vers le lavage de CO₂.

2. Procédé selon la revendication 1, **caractérisé en ce que**
g) le produit du fond du laveur d'eau est introduit directement dans la colonne de prédéshydratation.

3. Procédé selon la revendication 1 ou 2, **caractérisé en ce que** l'ensemble de l'éthylène alimenté, qui est introduit avant le réacteur dans le flux de gaz en circulation, est alimenté via le compresseur à jets.

4. Procédé selon la revendication 1 à 3, **caractérisé en ce que** le produit du fond du laveur d'eau est chauffé par échange de chaleur avec le gaz en circulation et est introduit dans la colonne de prédéshydratation.
